# EUROPEAN PATENT APPLICATION

(11) **EP 4 617 284 A1**
(43) Date of publication of application: **17.09.2025**
(21) Application number: 23887999.3
(22) Date of filing: 07.11.2023
(51) Int. Cl.: C07K 16/24, C07K 16/46, C12N 15/13, C12N 15/81, C12N 1/19

(54) **ANTI-IL-13 LONG-ACTING NANOBODY SEQUENCE AND USE THEREOF**

(30) Priority: 08.11.2022 CN 202211394330
(71) Applicant: Shanghai Novamab Biopharmaceuticals Co., Ltd., Pudong New Area, Shanghai 201321 (CN)
(72) Inventor: WAN, Yakun, Shanghai 201321 (CN); ZHU, Min, Shanghai 201321 (CN); GAI, Junwei, Shanghai 201321 (CN); LI, Guanghui, Shanghai 201321 (CN)
(74) Representative: Mewburn Ellis LLP
(86) International application number: PCT/CN2023/130165
(87) International publication number: WO 2024/099310

(57) **Abstract**

An anti-IL-13 long-acting nanobody, a coding sequence encoding the anti-IL-13 nano antibody, a corresponding expression vector, a host cell capable of expressing the nanobody, and a method for producing the nanobody.

## Description

### TECHNICAL FIELD

The present invention relates to the field of biomedical or biopharmaceutical technology, and more specifically to an anti-IL-13 long-acting nanobody sequence and applications thereof.

### BACKGROUND

Interleukin-13 (IL-13) is an important member of the interleukin family. It is a protein encoded by the IL-13 gene with a molecular weight of about 10 kD. IL-13 can be secreted by a variety of cells, such as CD4+ T cells, CD8+ T cells, mast cells, basophils, eosinophils and natural killer cells, especially type 2 helper T cells (Th2). IL-13 exerts diverse biological effects on mononuclear macrophages, B lymphocytes, NK cells and vascular endothelial cells, and others, serving as a mediator in the regulation of immune responses. In Th2 inflammation, Th2 cells produce a series of cytokines such as IL-4, IL-5, IL-9 and IL-13, among which IL-13 is considered to be the most central factor and is involved in almost all processes of Th2 inflammation. Numerous studies have proved that IL-13 plays a pivotal role in the pathogenesis of Th2 inflammation.

Multiple studies indicate that IL-13 is associated with autoimmune diseases related to Th2 immune response, such as asthma, atopic dermatitis (AD), chronic obstructive pulmonary disease (COPD), allergic rhinitis, inflammatory bowel disease, etc. As one of the targets for autoimmune diseases, IL-13 has been involved in clinical research related to biological agents, such as Adtralza (Tralokinumab, already marketed) from LEO Pharma, with investigational indications including atopic dermatitis, alopecia areata, asthma, ulcerative colitis, idiopathic pulmonary fibrosis, and chronic obstructive pulmonary disease; lebrikizumab (Phase III clinical trials) from Roche, with investigational indications including atopic dermatitis, eczema, asthma, Hodgkin's lymphoma, idiopathic pulmonary fibrosis, and chronic obstructive pulmonary disease; and Cendakimab (clinical phase II under study) from BMS, with investigational indications including eosinophilic esophagitis and asthma.

Among numerous factors, IL-13 and its signaling pathway have demonstrated their significant clinical value as promising therapeutic targets. Based on this, the use of IL-13 antagonists to block the biological activity of IL-13, which is expressed in large quantities in Th2-related diseases, should be a good method for improving Th2 inflammation. Nanobody, a naturally occurring heavy-chain antibody without a light chain, found in camelids, is currently the smallest known antigen-binding fragment. Antibody drug research based on its characteristic advantages has gradually attracted attention. Therefore, the development of nanobodies targeting IL-13 holds considerable clinical value, providing new therapeutic strategies for autoimmune diseases such as asthma and atopic dermatitis.

In summary, there remains a need in the art to develop a high-affinity and high-specificity long-acting nanobody against IL-13.

### SUMMARY OF THE INVENTION

The objective of the present invention is to provide a high-affinity and high-specificity anti-IL-13 long-acting nanobody.

Another objective of the present invention is to provide applications of the anti-IL-13 long-acting nanobody.

In a first aspect of the present invention, an anti-IL-13 nanobody is provided, wherein the complementary determining regions (CDRs) of the VHH chain in the nanobody are one or more selected from the group consisting of:
(1) CDR1 shown in SEQ ID NO: 2, CDR2 shown in SEQ ID NO: 4, and CDR3 shown in SEQ ID NO: 6; and
(2) CDR1 shown in SEQ ID NO: 11, CDR2 shown in SEQ ID NO: 13, and CDR3 shown in SEQ ID NO: 15.

In another preferred embodiment, the CDR1, CDR2 and CDR3 are separated by framework regions FR1, FR2, FR3 and FR4 of the VHH chain.

In another preferred embodiment, the VHH chain further comprises framework regions (FRs), and the framework regions (FRs) are one or more selected from the group consisting of:
(1) FR1 shown in SEQ ID NO: 1, FR2 shown in SEQ ID NO: 3, FR3 shown in SEQ ID NO: 5, and FR4 shown in SEQ ID NO: 7; and
(2) FR1 shown in SEQ ID NO: 10, FR2 shown in SEQ ID NO: 12, FR3 shown in SEQ ID NO: 14, and FR4 shown in SEQ ID NO: 16.

In another preferred embodiment, the CDR region of the nanobody VHH chain comprises an amino acid sequence having a sequence similarity of at least 80%, preferably at least 90%, more preferably at least 95%, even more preferably at least 99% to any one of SEQ ID NOs: 2, 4 and 6, SEQ ID NOs: 11, 13 and 15.

In another preferred embodiment, the amino acid sequence of the nanobody VHH chain CDR region comprises one or more amino acid substitutions, preferably conservative amino acid substitutions, compared to any one of SEQ ID NOs: 2, 4 and 6, SEQ ID NOs: 11, 13 and 15.

In another preferred embodiment, any one of the above amino acid sequences may further comprise a derivative sequence having one or more (e.g., 1-3, preferably 1-2, most preferably 1) amino acid additions, deletions, modifications, and/or substitutions while maintaining IL-13-specific binding capability.

In another preferred embodiment, the nanobody can specifically bind to IL-13.

In another preferred embodiment, the IL-13 is IL-13 from human or non-human mammals.

In another preferred embodiment, the IL-13 is IL-13 from humans, mice, rats, or non-human primates (such as monkeys).

In another preferred embodiment, the nanobody comprises a humanized antibody, a camelid-derived antibody, or a chimeric antibody.

In another preferred embodiment, the amino acid sequence of the VHH chain of the nanobody is selected from the group consisting of: SEQ ID NO: 8, SEQ ID NO: 17, and a combination thereof.

In another preferred embodiment, the anti-IL-13 nanobody comprises a monomer, a diabody (bivalent antibody), a tetrabody (tetravalent antibody), and/or a multibody (multivalent antibody).

In another preferred embodiment, the anti-IL-13 nanobody comprises one or more VHH chains having an amino acid sequence as shown in SEQ ID NO: 8 or SEQ ID NO: 17.

In another preferred embodiment, the anti-IL-13 nanobody comprises two VHH chains having an amino acid sequence as shown in SEQ ID NO: 8 or SEQ ID NO: 17.

In another preferred embodiment, the VHH chains are connected via a linking peptide.

In another preferred embodiment, the linking peptide is selected from the following sequences: (GaSb)x, wherein a, b, x=0 or 1 or 2 or 3 or 4 or 5 or 6 or 7 or 8 or 9 or 10 (preferably, a=4 and b=1, x=4).

In another preferred embodiment, the sequence of the linking peptide is shown in SEQ ID NO: 28.

In the second aspect of the present invention, an anti-IL-13 antibody is provided, which targets an IL-13 epitope and comprises the anti-IL-13 nanobody according to the first aspect of the invention.

In another preferred embodiment, the anti-IL-13 antibody comprises one or more anti-IL-13 nanobodies.

In another preferred embodiment, the anti-IL-13 antibody comprises a monomer, a diabody (bivalent antibody), a tetrabody (tetravalent antibody), and/or a multibody (multivalent antibody).

In another preferred embodiment, the anti-IL-13 antibody comprises one or more VHH chains having an amino acid sequence as shown in SEQ ID NO: 8 or SEQ ID NO: 17.

In another preferred embodiment, the anti-IL-13 antibody comprises two VHH chains having an amino acid sequence as shown in SEQ ID NO: 8 or SEQ ID NO: 17.

In another preferred embodiment, the antibody can specifically bind to IL-13.

In another preferred embodiment, the antibody can effectively block IL-13-induced SEAP release in HEK-Blue^{™} IL-4/IL-13 cells, demonstrating significantly superior blocking activity compared to the control antibody Lebrikizumab.

In another preferred embodiment, the antibody can effectively inhibit proliferation of HEK-Blue^{™} IL-4/IL-13 cells, demonstrating superior inhibitory activity compared to the control antibody 4B06.

In another preferred embodiment, the antibody is a nanobody.

In the third aspect of the present invention, a polynucleotide is provided, which encodes a protein selected from the following group: the anti-IL-13 nanobody according to the first aspect of the present invention or the anti-IL-13 antibody according to the second aspect of the present invention.

In another preferred embodiment, the polynucleotides are in a combined form.

In another preferred embodiment, the polynucleotide sequence comprises one or more sequences shown in SEQ ID NO: 9 or 18.

In another preferred embodiment, the polynucleotide includes RNA, DNA or cDNA.

In the fourth aspect of the present invention, an expression vector is provided, wherein the expression vector contains the polynucleotide according to the third aspect of the present invention.

In another preferred embodiment, the expression vector is selected from the group consisting of: DNA, RNA, a viral vector, a plasmid, a transposon, other gene transfer systems, and a combination thereof.

In another preferred embodiment, the expression vector includes a viral vector, such as a lentivirus, adenovirus, AAV virus, or retrovirus.

In the fifth aspect of the present invention, a host cell is provided, wherein the host cell contains the expression vector according to the fourth aspect of the present invention, or has the polynucleotide according to the third aspect of the present invention integrated into its genome.

In another preferred embodiment, the host cell includes a prokaryotic cell or a eukaryotic cell.

In another preferred embodiment, the host cell is selected from the group consisting of: *Escherichia coli,* yeast cells, mammalian cells, bacteriophages, and a combination thereof.

In another preferred embodiment, the prokaryotic cell is selected from the group consisting of: *Escherichia coli,* Bacillus subtilis, lactic acid bacteria, Streptomyces, Proteus mirabilis, and a combination thereof.

In another preferred embodiment, the eukaryotic cell is selected from the group consisting of: *Pichia pastoris,* Saccharomyces cerevisiae, Schizosaccharomyces pombe, Trichoderma, and a combination thereof.

In another preferred embodiment, the host cell is *Pichia pastoris.*

In the sixth aspect of the present invention, a method for producing an anti-IL-13 nanobody is provided, comprising the steps of:
(a) culturing the host cell according to the fifth aspect of the present invention under conditions suitable for producing nanobodies, thereby obtaining a culture containing the anti-IL-13 nanobody;
(b) isolating or recovering the anti-IL-13 nanobody from the culture; and
(c) optionally, purifying and/or modifying the anti-IL-13 nanobody obtained in step (b).

In the seventh aspect of the present invention, an immunoconjugate is provided, wherein the immunoconjugate comprises:
(a) the anti-IL-13 nanobody according to the first aspect of the present invention, or the anti-IL-13 antibody according to the second aspect of the present invention; and
(b) a conjugated moiety selected from the group consisting of: a detectable label, a drug, a toxin, a cytokine, a radionuclide, an enzyme, a gold nanoparticle/nanorod, a nanomagnetic particle, a viral coat protein or VLP, and a combination thereof.

In another preferred embodiment, the radionuclide includes:
(i) a diagnostic isotope selected from the group consisting of: Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, 1-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and a combination thereof.

In another preferred embodiment, the coupling moiety is a drug or a toxin.

In another preferred embodiment, the drug is a cytotoxic drug.

In another preferred embodiment, the cytotoxic drug is selected from the group consisting of: anti-tubulin drugs, DNA minor groove binding agents, DNA replication inhibitors, alkylating agents, antibiotics, folic acid antagonists, antimetabolites, chemosensitizers, topoisomerase inhibitors, vinca alkaloids, and a combination thereof.

In another preferred embodiment, examples of particularly useful cytotoxic drugs include, for example, DNA minor groove binding agents, DNA alkylating agents, and tubulin inhibitors, and typical cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4]benzodiazepines (PBDs), indolinobenzodiazepines and oxazolidinobenzodiazepines), vinca alkaloids, or a combination thereof.

In another preferred embodiment, the toxin is selected from the group consisting of: auristatins (e.g., auristatin E, auristatin F, MMAE and MMAF), chlortetracycline, maytansyl, ricin, ricin A-chain, combretastatin, duocarmycin, dolastatin, adriamycin, daunorubicin, paclitaxel, cisplatin, cc1065, ethidium bromide, mitomycin, etoposide, tenoposide, vincristine, vinblastine, colchicine, dihydroxyproline anthracnose dione, actinomycin, diphtheria toxin, Pseudomonas exotoxin (PE) A, PE40, abrin, abrin A chain, modeccin A chain, alpha-sarcin, gelonin, mitogellin, retstrictocin, phenomycin, enomycin, curicin, crotonin, calicheamicin, Sapaonaria officinalis inhibitor, glucocorticoids, and a combination thereof.

In another preferred embodiment, the coupling moiety is a detectable label.

In another preferred embodiment, the coupling moiety is selected from the group consisting of: fluorescent or luminescent labels, radioactive labels, MRI (magnetic resonance imaging) or CT (computer X-ray tomography) contrast agents, or enzymes capable of producing detectable products, radionuclides, biotoxins, cytokines (such as IL-2, etc.), antibodies, antibody Fc fragments, antibody scFv fragments, gold nanoparticles/nanorods, viral particles, liposomes, nanomagnetic particles, prodrug activating enzymes (for example, DT-diaphorase (DTD) or biphenyl hydrolase-like protein (BPHL)) and any form of nanoparticles.

In the eighth aspect of the present invention, a multispecific antibody is provided, wherein the multispecific antibody comprises: the anti-IL-13 nanobody according to the first aspect of the present invention, or the anti-IL-13 antibody according to the second aspect of the present invention.

In another preferred embodiment, the multispecific antibody further comprises an antibody Fc segment.

In the ninth aspect of the present invention, a recombinant protein is provided, wherein the recombinant protein has:
(i) the anti-IL-13 nanobody according to the first aspect of the present invention, or the anti-IL-13 antibody according to the second aspect of the present invention; and
(ii) an optional tag sequence assisting expression and/or purification.

In another preferred embodiment, the tag sequence includes an Fc tag, an HA tag and a 6His tag.

In another preferred embodiment, the recombinant protein specifically binds to the IL-13 protein.

In the tenth aspect of the present invention, a pharmaceutical composition is provided, wherein the pharmaceutical composition comprises:
(i) the anti-IL-13 nanobody according to the first aspect of the present invention, or the anti-IL-13 antibody according to the second aspect of the present invention, or the immunoconjugate according to the seventh aspect of the present invention, or the multispecific antibody according to the eighth aspect of the present invention or the recombinant protein according to the ninth aspect of the present invention; and
(ii) a pharmaceutically acceptable carrier.

In another preferred embodiment, the coupling moiety of the immunoconjugate is a drug, a toxin, and/or a therapeutic isotope.

In another preferred embodiment, the pharmaceutical composition further comprises other drugs for treating immune system diseases or tumors.

In another preferred embodiment, the other drugs for treating immune system diseases or tumors are selected from the group consisting of: budesonide, fluticasone, beclomethasone, mometasone furoate, salbutamol, theophylline, formoterol, tiotropium bromide, sulfasalazine, methotrexate, cyclophosphamide, fluorouracil, bleomycin, anastrozole, and a combination thereof.

In another preferred embodiment, the pharmaceutical composition is used to prepare drugs for preventing and/or treating IL-13-related diseases or symptoms.

In another preferred embodiment, the IL-13-related diseases or symptoms include immune system diseases.

In another preferred embodiment, the immune system diseases are selected from the group consisting of: asthma, atopic dermatitis (AD), chronic obstructive pulmonary disease (COPD), allergic rhinitis, inflammatory bowel disease, alopecia areata, ulcerative colitis, idiopathic pulmonary fibrosis, eczema, Hodgkin's lymphoma, idiopathic pulmonary fibrosis, eosinophilic esophagitis, and a combination thereof.

In the eleventh aspect of the present invention, a use of the anti-IL-13 nanobody according to the first aspect of the present invention, the anti-IL-13 antibody according to the second aspect of the present invention, the immunoconjugate according to the seventh aspect of the present invention, the multispecific antibody according to the eighth aspect of the present invention, the recombinant protein according to the ninth aspect of the present invention, or the pharmaceutical composition according to the tenth aspect of the present invention is provided, for:
(a) preparing drugs for preventing and/or treating IL-13-related diseases; and/or
(b) preparing reagents, assay plates, or kits for detecting IL-13.

In another preferred embodiment, the IL-13-related diseases or symptoms include immune system diseases.

In another preferred embodiment, the immune system diseases are selected from the group consisting of: asthma, atopic dermatitis (AD), chronic obstructive pulmonary disease (COPD), allergic rhinitis, inflammatory bowel disease, alopecia areata, ulcerative colitis, idiopathic pulmonary fibrosis, eczema, Hodgkin's lymphoma, idiopathic pulmonary fibrosis, eosinophilic esophagitis, and a combination thereof.

In another preferred embodiment, the IL-13 is human IL-13.

In another preferred embodiment, the reagent is a diagnostic reagent.

In another preferred embodiment, the diagnostic agent is a contrast agent.

In another preferred embodiment, the reagent is used to detect IL-13 protein or a fragment thereof in a sample.

In another preferred embodiment, the detection includes flow cytometry and cell immunofluorescence detection.

In another preferred embodiment, the use is diagnostic and/or non-diagnostic, and/or therapeutic and/or non-therapeutic.

In the twelfth aspect of the present invention, a method for detecting IL-13 protein in a sample is provided, wherein the method comprises the steps of:
(1) contacting the sample with the anti-IL-13 nanobody according to the first aspect of the present invention, or the anti-IL-13 antibody according to the second aspect of the present invention, or the immunoconjugate according to the seventh aspect of the present invention, or the multispecific antibody according to the eighth aspect of the present invention, or the recombinant protein according to the ninth aspect of the present invention;
(2) detecting whether an antigen-antibody complex is formed, wherein the formation of the complex indicates the presence of the IL-13 protein in the sample.

In another preferred embodiment, the method is a non-diagnostic and non-therapeutic method.

In the thirteenth aspect of the present invention, an IL-13 protein detection reagent is provided, wherein the detection reagent comprises:
(i) the anti-IL-13 nanobody according to the first aspect of the present invention, or the anti-IL-13 antibody according to the second aspect of the present invention, or the immunoconjugate according to the seventh aspect of the present invention, or the multispecific antibody according to the eighth aspect of the present invention or the recombinant protein according to the ninth aspect of the present invention; and
(ii) a diagnostically acceptable carrier.

In another preferred embodiment, the coupling moiety of the immunoconjugate is a diagnostic isotope.

In another preferred embodiment, the diagnostically acceptable carrier is a non-toxic, inert aqueous carrier medium.

In another preferred embodiment, the detection reagent is one or more of reagents selected from the group consisting of: isotope tracers, contrast agents, flow cytometry detection reagents, cell immunofluorescence detection reagents, nanomagnetic particles and imaging agents.

In another preferred embodiment, the detection reagent is used for *in vivo* detection.

In another preferred embodiment, the dosage form of the detection reagent is liquid or powder (such as aqueous solution, injection, lyophilized powder, tablet, buccal tablet, inhaler).

In the fourteenth aspect of the present invention, a kit for detecting IL-13 protein is provided, wherein the kit comprises the immunoconjugate according to the seventh aspect of the present invention or the detection reagent according to the thirteenth aspect of the present invention, and instructions.

In another preferred embodiment, the instructions state that the kit is used for non-invasively detecting the expression of IL-13 in a test subject.

In the fifteenth aspect of the present invention, a use of the immunoconjugate according to the seventh aspect of the present invention for preparing a contrast agent for *in vivo* detection of IL-13 protein is provided.

In another preferred embodiment, the detection is used for the diagnosis or prognosis of IL-13-related diseases or symptoms.

In the sixteenth aspect of the present invention, a method for treating IL-13-related diseases is provided, wherein the method comprises a step of administering the anti-IL-13 nanobody according to the first aspect of the present invention, the anti-IL-13 antibody according to the second aspect of the present invention, the immunoconjugate according to the seventh aspect of the present invention, the multispecific antibody according to the eighth aspect of the present invention, the recombinant protein according to the ninth aspect of the present invention, or the pharmaceutical composition according to the tenth aspect of the present invention to a subject in need thereof.

In another preferred embodiment, the subject comprises human or non-human mammals.

In another preferred embodiment, the non-human mammals comprise rodents (such as mice and rabbits) and non-human primates (such as monkeys).

It should be understood that, within the scope of the present invention, the above technical features of the present invention and the technical features specifically described hereinafter (such as in examples) may be combined with each other to accordingly constitute a new or preferred technical solution. For brevity, not all possible combinations are exhaustively described herein.

### BRIEF DESCRIPTION OF THE DRAWINGS

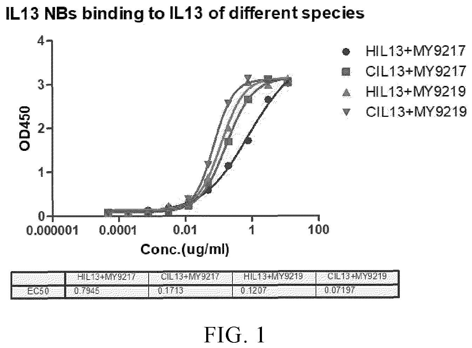

Figure 1 shows the ELISA method for detecting the binding activity of candidate antibodies to human IL-13 and cynomolgus monkey IL-13. The results show that MY9217 and MY9219 can simultaneously bind to human IL-13 and cynomolgus monkey IL-13.

### DETAILED DESCRIPTION

Through extensive and in-depth research, the inventors of the present invention have unexpectedly obtained anti-IL-13 nanobodies with excellent affinity and specificity after substantial screening.

Experimental results demonstrate that the nanobodies of the present invention exhibit outstanding functional activity, effectively blocking IL-13-induced SEAP release in HEK-Blue^{™} IL-4/IL-13 cells, with superior activity compared to control antibodies Lebrikizumab and 4B06. The nanobodies of the present invention possess favorable in vivo half-life and can effectively bind to human serum albumin, showing higher binding activity than Control Antibody 3 (HuNb3-11). The nanobodies of the present invention are capable of simultaneously binding to both human IL-13 and cynomolgus monkey IL-13. On this basis, the inventors have completed the present invention.

### Terms

As used herein, the term "binder" refers to a soluble receptor or a fragment or an analog thereof, or an antibody or a fragment or an analog thereof capable of binding to a target.

The "IL-13 binder" of the present invention refers to an antibody or a fragment or an analog thereof that can specifically recognize IL-13 and bind to the IL-13 antigen.

In order to more easily understand the present invention, some technical and scientific terms are specifically defined below. Unless otherwise clearly defined in this article, all other technical and scientific terms used herein have the meanings generally understood by those of ordinary skill in the art to which the present invention belongs. Before describing the present invention, it should be understood that the present invention is not limited to the specific methods and experimental conditions described, as such methods and conditions may vary. It should also be understood that the terms used herein are intended only to describe specific embodiments, and are not intended to be restrictive, and the scope of the present invention will be limited only by the appended claims.

Unless otherwise defined, all technical and scientific terms used herein have the same meaning as commonly understood by those of ordinary skill in the art to which the invention belongs. As used herein, the term "about" when used in reference to a specific recited value means that the value may vary by no more than 1% from the recited value. For example, as used herein, the expression "about 100" includes 99 and 101 and all values therebetween (e.g., 99.1, 99.2, 99.3, 99.4, etc.).

As used herein, the term "optional" or "optionally" means that the event or situation described subsequently may occur but does not have to occur. For example, "optionally comprising 1 to 3 antibody heavy-chain variable regions" refers to that a particular sequence may have but not necessarily have 1, 2 or 3 antibody heavy-chain variable regions.

### Interleukin-13 (IL-13)

Interleukin-13 (IL-13) is an important member of the interleukin family. It is a protein encoded by the IL-13 gene with a molecular weight of about 10 kD. IL-13 exerts diverse biological effects on mononuclear macrophages, B lymphocytes, NK cells and vascular endothelial cells, and others, serving as a mediator in the regulation of immune responses.

### Antibody

As used herein, the term "antibody" or "immunoglobulin" refers to a heterotetrameric glycoprotein of about 150,000 Daltons with identical structural features, each composed of two identical light chains (L) and two identical heavy chains (H). Each light chain is linked to a heavy chain by one covalent disulfide bond, while the number of disulfide bonds between heavy chains varies among different immunoglobulin isotypes. Each heavy chain and light chain additionally has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Specific amino acid residues form an interface between the variable regions of the light and heavy chains.

As used herein, the terms "single domain", "VHH", "nanobody", "heavy-chain antibody" (single domain antibody, sdAb, or nanobody) have the same meaning and are used interchangeably, referring to the cloned variable region of an antibody heavy chain that constructs a nanobody (VHH) consisting solely of a single heavy chain variable region, which represents the smallest functional antigen-binding fragment. Usually, an antibody naturally lacking the light-chain and heavy-chain constant region 1 (CH1) is first obtained, and then the variable region of the antibody heavy chain is cloned to construct a nanobody (VHH) composed exclusively of a single heavy-chain variable region.

As used herein, the term "antigen-binding fragment" refers to a Fab fragment, a Fab' fragment, a F(ab')2 fragment, or a single Fv fragment, having an antigen-binding activity. The Fv antibody contains a heavy-chain variable region and a light-chain variable region, but does not have a constant region, and has a minimum antibody fragment with all antigen binding sites. Generally, the Fv antibody further contains a polypeptide linker between the VH and VL domains, and can form a structure required for antigen binding.

As used herein, the term "antigenic determinant" refers to three-dimensional spatial sites distributed discontinuously on an antigen and identified by the antibody or antigen-binding fragment of the present invention.

The present invention includes not only intact antibodies, but also fragments of immunocompetent antibodies or fusion proteins formed by antibodies with other sequences Therefore, the present invention further comprises fragments, derivatives, and analogues of the antibody.

In the present invention, the antibody comprises murine, chimeric, humanized or fully human antibodies prepared with techniques familiar to those skilled in the art. Recombinant antibodies, for example, chimeric and humanized monoclonal antibodies (including human and non-human moieties), can be prepared using the recombinant DNA techniques well known in the art.

As used herein, the term "monoclonal antibody" refers to an antibody secreted by a clone derived from a single cell source. The monoclonal antibody is highly specific for a single epitope. The cells may be eukaryotic, prokaryotic, or phage clonal cell lines.

As used herein, the term "chimeric antibody" is an antibody molecule expressed by splicing the V region gene of a murine antibody and the C region gene of a human antibody into a chimeric gene, and then inserting the chimeric gene into a vector to transfect a host cell. It not only retains the high specificity and affinity of parental mouse antibody, but also enables its human-derived Fc segment to effectively mediate the biological effector functions.

As used herein, the term "humanized antibody" is a variable region modification form of the murine antibody of the present invention. It has CDRs derived from (or substantially derived from) a non-human antibody (preferably a mouse monoclonal antibody), and FRs and constant regions substantially derived from the human antibody sequence. That is, the CDR sequences of the murine antibody are grafted onto different types of human germline antibody framework sequences. Since the CDR sequences are responsible for most of the antibody-antigen interactions, recombinant antibodies that mimic the properties of specific naturally-occurring antibodies can be expressed by constructing expression vectors.

In the present invention, the antibody may be monospecific, bispecific, trispecific, or possess higher orders of multispecificity.

As used herein, the term "variable" indicates that certain portions of the antibody's variable regions differ in sequence, conferring binding capacity and specificity to particular antigens. However, variability is not evenly distributed throughout the antibody variable regions. It is concentrated in three segments, known as complementarity determining regions (CDRs) or hypervariable regions, in the light-chain and heavy-chain variable regions. The more conservative portions of the variable regions are known as framework regions (FRs). The four FRs in the natural heavy-chain and light-chain variable regions are roughly in a b-folded configuration linked by three CDRs that form a linking ring, and in some cases, the four FRs may form a partially b-folded structure. The CDRs in each chain are closely brought together by the FRs, and, together with the CDRs from another chain, form an antigen-binding site of the antibody (see Kabat et al., NIH Publ. No. 91-3242, Volume I, page 647-669 (1991)). The constant regions do not directly participate in antibody-antigen binding but exhibit various effector functions, such as mediating antibody-dependent cellular cytotoxicity.

As known to those skilled in the art, immunoconjugates and fusion expression products include: conjugates formed by linking drugs, toxins, cytokines, radionuclides, enzymes, and other diagnostic or therapeutic molecules to the antibody or its fragments of the present invention. The present invention also includes cell surface markers or antigens that bind to the anti-IL-13 antibodies or fragments thereof.

As used herein, the term "heavy-chain variable region" is interchangeable with "VH".

As used herein, the term "variable region" is interchangeable with "complementarity determining region (CDR)".

In a preferred embodiment of the present invention, the heavy-chain variable region of the antibody comprises three complementarity determining regions: CDR1, CDR2, and CDR3.

In a preferred embodiment of the present invention, the heavy chain of the antibody comprises the above-mentioned heavy-chain variable region and heavy-chain constant region.

In the present invention, the terms "antibodies of the present invention", "proteins of the present invention", or "polypeptides of the present invention" are used interchangeably, all referring to polypeptides that specifically bind to IL-13 proteins, such as proteins or polypeptides having a heavy-chain variable region. They may or may not contain an initial methionine.

The present invention further provides other proteins or fusion expression products having the antibodies of the present invention. Specifically, the present invention includes any protein or protein conjugate and fusion expression product (i.e., immunoconjugate and fusion expression product) having a heavy chain containing a variable region, as long as the variable region is identical to or at least 90% homologous to the heavy-chain variable region of the antibodies of the present invention, preferably at least 95% homologous.

Generally, the antigen binding properties of an antibody can be described by three specific regions located in the variable region of the heavy chain, termed variable regions (CDRs). This segment is divided into four framework regions (FRs). The amino acid sequences of the four FRs are relatively conservative and do not directly participate in the binding reaction. These CDRs form a ring structure, and the β-folds formed by the FRs in between are close to each other in spatial structure. The CDRs on the heavy chain and the CDRs on the corresponding light chain constitute the antigen binding site of the antibody. It is possible to determine which amino acids make up the FRs or CDRs by comparing the amino acid sequences of antibodies of the same type.

The variable regions of the heavy chains of the antibodies of the present invention are of particular interest because they are at least partially involved in antigen binding. Therefore, the present invention includes molecules having antibody heavy-chain variable regions with CDRs, as long as their CDRs share more than 90% (preferably more than 95%, and most preferably more than 98%) homology with the CDRs identified herein.

The present invention includes not only intact antibodies, but also fragments of immunocompetent antibodies or fusion proteins formed by antibodies with other sequences Therefore, the present invention further comprises fragments, derivatives, and analogues of the antibody.

As used herein, the terms "fragment", "derivative" and "analog" refer to polypeptides that substantially retain the same biological function or activity as the antibodies of the present invention. The polypeptide fragments, derivatives or analogs of the present invention may be (i) polypeptides in which one or more conservative or non-conservative amino acid residues (preferably conservative amino acid residues) are substituted, and such substituted amino acid residues may or may not be encoded by the genetic code, or (ii) polypeptides having substituent groups in one or more amino acid residues, or (iii) polypeptides formed by fusion of a mature polypeptide with another compound (such as a compound that prolongs the half life of the polypeptide, such as polyethylene glycol), or (iv) polypeptides formed by fusion of an additional amino acid sequence to the polypeptide sequence (such as a leader sequence or secretory sequence or a sequence or proprotein sequence used to purify the polypeptide, or a fusion protein formed with a 6His tag). As will be appreciated by those skilled in the art based on the teachings herein, such fragments, derivatives, and analogs fall within the scope of common knowledge in the art.

The antibodies of the present invention refer to polypeptides having IL-13 binding activity and comprising the above-mentioned CDR regions. The term also includes variant forms of polypeptides comprising the above-mentioned CDR regions and having the same function as the antibodies of the present invention. These variant forms include (but are not limited to): Deletions, insertions, and/or substitutions of one or more amino acids (typically 1-50, preferably 1-30, more preferably 1-20, and most preferably 1-10 amino acids), and addition of one or several (typically fewer than 20, preferably fewer than 10, more preferably fewer than 5) amino acids at the C-terminus and/or N-terminus. For example, in the art, substitution with amino acids having similar properties generally does not alter protein function Similarly, addition of one or a few amino acids at the C-terminus and/or N-terminus typically does not affect protein function. This term further includes active fragments and active derivatives of the antibodies of the present invention.

Variant forms of the polypeptide include: homologous sequences, conservative variants, allelic variants, natural mutants, induced mutants, proteins encoded by DNA that can hybridize with the encoding DNA of the antibody of the present invention under high or low stringency conditions, and polypeptides or proteins obtained using antiserum against the antibody of the present invention.

The invention also provides other polypeptides, such as fusion proteins comprising nanobodies or fragments thereof. In addition to almost full-length polypeptides, the present invention also includes fragments of nanobodies of the invention. Typically, such fragments comprise at least about 50 contiguous amino acids (preferably at least about 50, more preferably at least about 80, and most preferably at least about 100 contiguous amino acids) of the antibodies of the present invention.

In the present invention, "conservative variants of the antibodies of the present invention" refer to polypeptides formed by replacing up to 10, preferably up to 8, more preferably up to 5, and most preferably up to 3 amino acids with amino acids having similar or similar properties compared to the amino acid sequence of the antibodies of the present invention. Such conservative variant polypeptides are preferably produced by amino acid substitutions according to Table A.

**Table A**

| Initial residue | Representative substituent | Preferred substituent |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Encoding nucleic acid and expression vector

The present invention further provides a polynucleotide molecule encoding the above-mentioned antibody, its fragment or its fusion protein. The polynucleotide of the present invention can be in the form of DNA or RNA. The DNA form includes cDNA, genomic DNA or artificially synthesized DNA. The DNA can be single-stranded or doublestranded. The DNA can be a coding strand or a non-coding strand.

The polynucleotide encoding the mature polypeptide of the present invention includes: a coding sequence encoding only a mature polypeptide; a coding sequence of a mature polypeptide and various additional coding sequences; a coding sequence of a mature polypeptide (and optionally additional coding sequences) and non-coding sequences.

The term "polynucleotide encoding a polypeptide" may include a polynucleotide encoding the polypeptide, as well as those that additionally contain extra coding and/or non-coding sequences.

The present invention also relates to polynucleotides that hybridize to the above-mentioned sequences and share at least 50% (preferably at least 70%, more preferably at least 80%) sequence identity. The present invention particularly relates to polynucleotides that can hybridize to the polynucleotides of the present invention under stringent conditions. In the present invention, "stringent conditions" refer to: (1) hybridization and elution at lower ionic strength and higher temperature, such as 0.2×SSC, 0.1% SDS, 60°C; or (2) the addition of denaturing agents during hybridization, such as 50% (v/v) formamide, 0.1% calf serum/0.1% Ficoll, 42°C, etc.; or (3) hybridization occurs only when the identity between the two sequences is at least 90%, preferably at least 95%. In addition, the polypeptide encoded by the hybridizable polynucleotide must retain the same biological function and activity as the mature polypeptide.

The full-length nucleotide sequences of the antibodies of the present invention or their fragments can usually be obtained by PCR amplification, recombination or artificial synthesis. A feasible method is to synthesize the relevant sequence by artificial synthesis, especially when the fragment is short. Generally, a fragment with a very long sequence may be obtained by first synthesizing multiple small fragments, and then linking these small fragments. In addition, the coding sequence of the heavy chain and the expression tag (such as 6His) can be fused together to form a fusion protein.

Once relevant sequences are obtained, they may be obtained on a large scale by recombination. This is generally done by cloning them into vectors, then transferring them into cells, and then isolating the relevant sequences from the proliferated host cell by means of a conventional method. The biomolecules (nucleic acids, proteins, etc.) involved in the present invention include biomolecules in isolated form.

At present, the DNA sequence encoding the protein of the present invention (or its fragment, or its derivative) can be obtained completely by chemical synthesis. The DNA sequence can then be introduced into various existing DNA molecules (or vectors) and cells known in the art. In addition, mutations can also be introduced into the protein sequence of the present invention by chemical synthesis.

The present invention further relates to vectors comprising the aforementioned appropriate DNA sequences and appropriate promoters or control sequences. These vectors may be used to transform appropriate host cells to enable them to express proteins.

The host cells may be prokaryotic cells, such as bacterial cells; or lower eukaryotic cells, such as yeast cells; or higher eukaryotic cells, such as mammalian cells. Representative examples include: *Escherichia coli,* Streptomyces; bacterial cells of Salmonella typhimurium; fungal cells such as yeast; insect cells of Drosophila S2 or Sf9; animal cells such as CHO, COS7, 293 cells.

Transformation of host cells with recombinant DNA can be carried out using conventional techniques well known to those skilled in the art. When the host is a prokaryotic organism such as *Escherichia coli,* competent cells that can absorb DNA can be harvested after the exponential growth phase and treated with the CaCl2 method, the steps used are well known in the art. Another method is to use MgCl2. If necessary, transformation can also be carried out using electroporation. When the host is a eukaryotic organism, the following DNA transfection methods can be selected: calcium phosphate co-precipitation, conventional mechanical methods such as microinjection, electroporation, lipid-based encapsulation.

The obtained transformants can be cultured using standard methods to express polypeptides encoded by the genes of the present invention. The culture medium selection depends on the host cell type and may include any conventional medium suitable for maintaining cell growth under optimal conditions. Upon reaching appropriate cell density, selected promoters are induced through suitable methods (e.g., temperature shift or chemical induction), followed by additional cultivation.

The recombinant polypeptides produced via these methods may be expressed intracellularly, on cell membranes, or secreted extracellularly. Where necessary, the recombinant proteins may be isolated and purified using various techniques that exploit their physical, chemical, or other properties. These methods are well known to those skilled in the art. Examples of these methods include but are not limited to: conventional renaturation treatment, treatment with protein precipitants (salting out method), centrifugation, osmotic sterilization, ultra-treatment, ultracentrifugation, molecular sieve chromatography (gel filtration), adsorption chromatography, ion exchange chromatography, high performance liquid chromatography (HPLC) and other various liquid chromatography techniques and combinations of these methods.

The antibodies of the invention may be used alone or in combination or conjugated to a detectable label (for diagnostic purposes), a therapeutic agent, a PK (protein kinase) modifying moiety, or any combination of these.

Detectable labels for diagnostic purposes include, but are not limited to, fluorescent or luminescent labels, radioactive labels, MRI (magnetic resonance imaging) or CT (computed X-ray tomography) contrast agents, or enzymes capable of producing a detectable product.

Therapeutic agents that can be combined or coupled with the antibodies of the present invention include, but are not limited to: 1. radionuclides; 2. biological toxins; 3. cytokines such as IL-2; 4. gold nanoparticles/nanorods; 5. viral particles; 6. liposomes; 7. nanomagnetic particles; 8. prodrug activating enzymes (e.g., DT-diaphorase (DTD) or biphenyl hydrolase-like protein (BPHL)), etc.

### Anti-IL-13 nanobody

As used herein, the terms "nanobody of the present invention", "nanobodies of the present invention", "anti-IL-13 nanobody of the present invention", "IL-13 nanobody of the present invention", "anti-IL-13 nanobody", and "IL-13 nanobody" have the same meaning and are used interchangeably, all referring to nanobodies that specifically recognize and bind to IL-13 (including human IL-13).

As used herein, the term "IL-13" refers to the IL-13 protein, whose amino acid sequence is shown in SEQ ID No: 1.

In a preferred embodiment of the present invention, the anti-IL-13 nanobody comprises a monomer, a diabody (bivalent antibody), a tetrabody (tetravalent antibody), and/or a multibody (multivalent antibody).

Typically, the anti-IL-13 nanobody comprises two VHH chains having the amino acid sequences shown in SEQ ID NO: 8 and SEQ ID NO: 17.

In another preferred embodiment, the VHH chains are connected via a linking peptide.

In another preferred embodiment, the linking peptide is selected from the following sequences: (GaSb)x, wherein a, b, x=0 or 1 or 2 or 3 or 4 or 5 or 6 or 7 or 8 or 9 or 10 (preferably, a=4 and b=1, x=4).

In another preferred embodiment, the sequence of the linking peptide is GGGGSGGGGSGGGGSGGGGS.

In another preferred embodiment, the CDR region of the nanobody VHH chain comprises an amino acid sequence having a sequence similarity of at least 80%, preferably at least 90%, more preferably at least 95%, even more preferably at least 99% to any one of SEQ ID NOs: 2, 4 and 6, SEQ ID NOs: 11, 13 and 15.

In another preferred embodiment, the amino acid sequence of the nanobody VHH chain CDR region comprises one or more amino acid substitutions compared to a sequence similarity 11, 13 and 15, preferably conservative amino acid substitutions.

### Labeled nanobodies

In a preferred embodiment of the present invention, the nanobody carries a detectable label. More preferably, the label is selected from the group consisting of: an isotope, a colloidal gold label, a colored label or a fluorescent label.

Colloidal gold labeling can be performed using methods known to those skilled in the art. In a preferred embodiment of the present invention, the IL-13 nanobody is labeled with colloidal gold to obtain a colloidal gold labeled nanobody.

### Preparation of antibodies

Any method suitable for producing an antibody can be used to produce the IL-13 nanobody of the present application. For example, animals can be immunized with linked or natural IL-13 or fragments thereof. Suitable immunization methods may be used, including adjuvants, immunostimulants, and repeated booster immunizations, and one or more routes may be used.

Any suitable form of IL-13 may be used as an immunogen (antigen) to produce a non-human antibody specific to IL-13 and to screen the biological activities of the antibody. The stimulating immunogen can be recombinant IL-13 or a fragment thereof. The immunogen may be used alone, or in combination with one or more immunogenicity enhancers known in the art. The immunogen may be purified from a natural source, or produced in a genetically modified cell. An DNA encoding the immunogen may be genomic or non-genomic (for example, cDNA) in origin. A suitable genetic vector may be used to express the DNA encoding the immunogen, and the vector comprises, but is not limited to: an adenovirus vector, an adeno-associated virus vector, a baculovirus vector, a material, and a non-viral vector.

An exemplary method for producing the anti-IL-13 antibody of the present application is described in Example 1.

The antibody of the present invention can be selected any class of immunoglobulins, including IgG and IgE. Preferably, the antibody is an IgG antibody, for example IgG1 subtype. With the biological assays described in the examples below, it is easy to optimize an essential constant domain sequence by screening antibodies, so as to produce the desired bioactivity.

Similarly, any type of light chain can be used in the compounds and methods herein. Specifically, κ and λ chains or their variants may be used in the compounds and methods of the present invention.

The sequence of the DNA molecule of the antibody or the fragment thereof in the present invention can be obtained by conventional techniques, such as methods using PCR amplification or genomic library screening and the like. In addition, the coding sequences of the light and heavy chains may also be fused together to form a single-chain antibody.

Once relevant sequences are obtained, they may be obtained on a large scale by recombination. This is generally done by cloning them into vectors, then transferring them into cells, and then isolating the relevant sequences from the proliferated host cell by means of a conventional method.

In addition, the relevant sequences may also be synthesized by using an artificial synthesis method, in particular when a fragment is short. Generally, a fragment with a very long sequence may be obtained by first synthesizing multiple small fragments, and then linking these small fragments. The DNA sequence can then be introduced into various existing DNA molecules (or vectors) and cells known in the art.

The present invention further relates to vectors comprising the aforementioned appropriate DNA sequences and appropriate promoters or control sequences. These vectors may be used to transform appropriate host cells to enable them to express proteins.

The host cells may be prokaryotic cells, such as bacterial cells; or lower eukaryotic cells, such as yeast cells; or higher eukaryotic cells, such as mammalian cells. The preferred animal cells comprise (but are not limited to): CHO-S, CHO-K1, and HEK-293 cells.

The step of transforming the host cells with recombinant DNAs in the present invention may be performed using techniques well known in the art. A resulting transformant may be cultured by a conventional method, and it expresses the polypeptide encoded by the gene of the present invention. According to the host cells used, they are cultured in a conventional medium under suitable conditions.

Usually, the host cells are cultured and transformed under conditions suitable for the expression of the antibody of the present invention. Then, the antibody of the present invention is purified and obtained using conventional immunoglobulin purification steps, such as protein A-Sepharose, hydroxyapatite chromatography, gel electrophoresis, dialysis, ion exchange chromatography, hydrophobic chromatography, molecular sieve chromatography, or affinity chromatography, and other conventional separation and purification means well known to those skilled in the art.

The resulting monoclonal antibody may be identified by a conventional means. For instance, the binding specificity of the monoclonal antibody may be determined by immunoprecipitation or *in vitro* binding assays, such as radioimmunoassay (RIA) or enzyme-linked immunosorbent assay (ELISA).

### Pharmaceutical composition and application

The present invention further provides a pharmaceutical composition. Preferably, the composition is a pharmaceutical composition, which comprises the antigen-binding protein described above, or an active fragment thereof, or a fusion protein thereof, and a pharmaceutically acceptable carrier. Usually, these substances may be formulated in a non-toxic, inert, and pharmaceutically acceptable aqueous carrier medium, of which the pH is usually about 5-8, preferably about 6-8, although the pH value may vary with the properties of the formulated substances and the symptoms to be treated. The formulated pharmaceutical composition can be administered by conventional routes, including (but not limited to): intravenous injection, intravenous drip, subcutaneous injection, local injection, intramuscular injection, intratumor injection, intraperitoneal injection (such as intraperitoneal), intracranial injection, or intracavitary injection.

In the present invention, the term "pharmaceutical composition" refers to a composition wherein the bispecific antibody of the present invention is combined with a pharmaceutically acceptable carrier to form a pharmaceutical formulation for more stable therapeutic efficacy. These formulations can maintain the conformational integrity of the core amino acid sequence of the disclosed bispecific antibody while protecting the multifunctional groups of the protein from degradation (including but not limited to aggregation, deamidation, or oxidation).

The pharmaceutical composition of the present invention contains a safe and effective amount (such as 0.001-99 wt%, preferably 0.01-90 wt%, more preferably 0.1-80 wt%) of the above-mentioned bispecific antibody of the present invention (or its conjugate) and a pharmaceutically acceptable carrier or excipient. Such carriers include (but are not limited to): saline, buffer, glucose, water, glycerol, ethanol, and a combination thereof. A pharmaceutical preparation should match the mode of administration. The pharmaceutical composition of the present invention may be prepared into an injection form, for example, by means of a conventional method using normal saline or an aqueous solution containing glucose and other adjuvants. The pharmaceutical composition such as an injection and a solution should be manufactured under an aseptic condition. The dosage of an active ingredient is a therapeutically effective amount, for example, about 10µg/kg body weight to about 50 µg/kg body weight per day. Furthermore, the bispecific antibody of the present invention may also be used together with other therapeutic agents.

When in use, the bispecific antibody or the immunoconjugate thereof are administered to mammals at a safe and effective amount, which is usually at least about 10 µg/kg body weight and in most cases, does not exceed about 50 mg/kg body weight, preferably the dosage is about 10 µg/kg body weight to about 10mg/kg body weight. Without doubt, the specific dosage should also consider factors such as the route of administration and the health condition of a patient, which are within the scope of skills of a proficient physician.

### Detection method

The present invention also relates to a method for detecting IL-13 protein. The method generally comprises the following steps: obtaining a cell and/or tissue sample; dissolving the sample in a medium; and detecting the level of IL-13 protein in the dissolved sample.

In the detection method of the present invention, the sample used is not particularly limited, and a representative example is a cell containing sample present in a cell preservation solution.

### Detection uses and kit

The antibodies of the present invention can be used in detection applications, for example, for detecting a sample to provide diagnostic information.

In the present invention, the sample (specimen) used includes cells, tissue samples and biopsy specimens. The term "biopsy" used in the present invention includes all types of biopsies known to those skilled in the art. Therefore, the biopsy used in the present invention can include, for example, tissue samples prepared by endoscopic methods or puncture or needle biopsy of an organ.

Samples used in the present invention include fixed or preserved cell or tissue samples.

The present invention also provides a kit containing the antibodies (or fragments thereof) of the present invention. In a preferred embodiment of the present invention, the kit further comprises a container, instructions for use, a buffer, etc. In a preferred embodiment, the antibody of the present invention can be fixed to an assay plate.

### Application

As described above, the nanobodies of the present invention have a wide range of biological and clinical application values, and their applications involve the diagnosis and treatment of IL-13-related diseases or symptoms, basic medical research, biological research, and other fields. A preferred application is for clinical diagnosis and targeted therapy of IL-13.

### The main advantages of the present invention include:

(a) The nanobody of the present invention exhibits excellent functional activity, with superior performance compared to the control antibody Lebrikizumab.
(b) The nanobody of the present invention effectively blocks IL-13-induced SEAP release in HEK-Blue^{™} IL-4/IL-13 cells, demonstrating higher activity than the control antibody Lebrikizumab.
(c) The nanobody of the present invention can be produced using a microbial expression system, resulting in low production costs.

The present invention will be further described below in conjunction with specific examples. It should be understood that these examples are intended to illustrate the present invention only and are not intended to limit the scope of the present invention. The experimental methods for which specific conditions are not specified in the following examples are generally performed under conventional conditions, such as those described in Sambrook et al., Molecular Cloning: A Laboratory Manual (New York: Cold Spring Harbor Laboratory Press, 1989), or under conditions recommended by the manufacturer. Unless otherwise specified, percentages and parts are calculated by weight.

Unless otherwise specified, the materials and reagents used in the examples of the present invention are commercially available products.

### Example 1 Immune library construction and screening

High-purity IL-13-Fc protein was mixed with immunoadjuvant and used to immunize Xinjiang Bactrian camels. After seven immunizations, peripheral blood was collected from the camels to measure serum antibody titers. Immunization was considered successful when the antibody titer exceeded 1: 1000. Peripheral blood mononuclear cells (PBMCs) were isolated from camel peripheral blood, and total RNA was extracted. The RNA was reverse-transcribed into cDNA, and VHH fragments were amplified by nested PCR. The amplified fragments were then cloned into a phage display vector and transfected into TG1 cells to construct a phage-displayed nanobody library. Through three rounds of "adsorption-washing-elution" cycles, IL-13-specific nanobody phages were enriched. A total of 600 randomly selected monoclonal clones were screened by ELISA of the supernatant.

Finally, 39 nanobodies with different CDR3 region sequences were obtained.

### Example 2 Screening of blocking nanobodies using HEK-Blue^{™} IL-4/IL-13 cell activity assay

The above 39 nanobodies were purified from *Escherichia coli* strains to obtain high-purity nanobodies, and the functional activity of the nanobodies was detected using HEK-Blue^{™} IL-4/IL-13 cells (purchased from InvivoGen). HEK-Blue^{™} IL-4/IL-13 cells produced SEAP under IL-13 stimulation, and the level of STAT6-induced SEAP in the supernatant was determined using QUANTI-Blue^{™} Solution. The stimulation of HEK-Blue^{™} IL-4/IL-13 cells by recombinant human IL-13 can be blocked by neutralizing IL-13 nanobodies. The functional activity of the above 39 candidate nanobodies was detected using this method.

HEK-Blue^{™} IL-4/IL-13 cells were resuspended in DMEM medium containing 1% FBS, counted and dispensed into 96-well plates, and pre-mixtures containing IL-23 nanobody and IL-13 protein were added and cultured in an incubator for 24 hours. The cultured cell supernatant was removed, and nine times the amount of QUANTI-Blue^{™} reagent was added and incubated at 37°C for 3 hours. The absorbance at 620nm was detected by an enzyme reader.

The results are shown in Table 1, where the control VHH1 is the nanobody sequence derived from 4B06 in patent WO2021116182A1.

**Table 1 Blocking activity of monovalent nanobodies**

| **Antibody ID** | **IC₅₀ (µg/mL)** | **Antibody ID** | **IC₅₀ (µg/mL)** | **Antibody ID** | **IC₅₀ (µg/mL)** |
|---|---|---|---|---|---|
| Nb1-83 | NA | Nb5-37 | NA | Nb4-25 | NA |
| Nb8-76 | 0.1278 | Nb5-62 | NA | Nb4-69 | NA |
| Nb10-50 | 0.1743 | Nb6-53 | NA | Nb4-78 | NA |
| Nb10-51 | NA | Nb10-17 | 0.9818 | Nb6-57 | NA |
| Nb1-52 | NA | Nb31-36 | 0.9923 | Nb2-42 | 0.2219 |
| Nb1-78 | NA | Nb8-73 | NA | Nb10-1 | 0.7031 |
| Nb2-20 | NA | Nb10-48 | NA | Nb10-5 | 0.4634 |
| Nb2-88 | 0.4450 | Nb1-45 | NA | Nb10-44 | 0.4196 |
| Nb3-23 | NA | Nb1-69 | NA | Nb16-4 | 0.1167 |
| Nb3-68 | 0.7476 | Nb2-37 | NA | Nb22-63 | 0.2693 |
| Nb3-94 | NA | Nb5-53 | NA | Nb22-8 | 0.2182 |
| Nb4-11 | 0.4825 | Nb3-58 | NA | Nb25-58 | 0.0110 |
| Nb4-81 | NA | Nb4-12 | NA | Nb25-59 | 0.1417 |
| Control VHH | 1.021 | | | | |

### Example 3 Activity evaluation of humanized nanobodies

Nanobodies demonstrating favorable cellular activity were subjected to humanization design, following the method described in Example 4 of Chinese Patent CN2018101517526. For each nanobody, the framework region sequences were humanized while maintaining the variable regions unchanged. The humanized nanobody sequences were then synthesized into the yeast expression vector pPICZaA using *Pichia pastoris* codon-optimized nucleotide sequences. The recombinant plasmids containing nanobody genes were linearized and electroporated into *Pichia pastoris* X33 competent cells. Single clones were then selected from antibiotic plates, and nanobody expression was induced with methanol before measuring antibody levels in the culture medium. The detection results are shown in Table 2.

Simultaneously, nanobodies were purified from *Pichia pastoris* fermentation supernatants for functional activity assessment using HEK-Blue^{™} IL-4/IL-13 cells.

As shown in Table 2, eight humanized nanobodies exhibited excellent functional activity.

**Table 2 Humanized nanobody production and activity evaluation**

| **ID before humanization** | **ID after humanization** | **Yeast cell expression production (g/L)** | **Humanized nanobody IC₅₀ (µg/mL)** | **Control nanobody IC₅₀ (µg/mL)** |
|---|---|---|---|---|
| Nb8-76 | MY9280 | 0.5 | / | / |
| Nb10-50 | MY9281 | 4.0 | Activity loss | 0.020 |
| Nb2-88 | MY9282 | 1.0 | Activity loss | 0.020 |
| Nb3-68 | MY9283 | 0 | / | / |
| Nb4-11 | MY9284 | 0.2 | / | / |
| Nb10-17 | MY9285 | 1.0 | 4.721 | 0.020 |
| Nb31-36 | MY9286 | 4.0 | 3.423 | 0.020 |
| Nb2-42 | MY9287 | 4.0 | 1.006 | 0.022 |
| Nb10-1 | MY9288 | 4.0 | Activity loss | 0.021 |
| Nb10-5 | MY9289 | 2.0 | Activity loss | 0.021 |
| Nb16-4 | MY9290 | 2.0 | 0.440 | 0.021 |
| Nb22-63 | MY9291 | 4.0 | 1.069 | 0.021 |
| Nb22-8 | MY9292 | 4.0 | 2.745 | 0.023 |
| Nb25-58 | MY9293 | 6.0 | 1.049 | 0.022 |
| Nb25-59 | MY9295 | 1.0 | 0.089 | 0.022 |
| Nb10-44 | MY9297 | 0.5 | / | / |

### Example 4 Construction and expression of bivalent nanobodies

To further enhance nanobody activity, the eight humanized nanobodies were pairwise combined to form bivalent constructs, resulting in a total of 64 bivalent nanobodies. Each pair of monomers was connected by a GS20 linker (amino acid sequence such as SEQ ID NO: 28). The diabody base sequences optimized by *Pichia pastoris* codon were cloned into the pPICZaA vector. The linearized recombinant plasmids were electroporated into *Pichia pastoris* X33 competent cells. Single clones were then selected from antibiotic plates, and antibody expression was induced with methanol before measuring antibody levels in the culture medium.

Through yield evaluation, 11 candidate bivalent nanobodies showed production levels exceeding 2 g/L, as presented in Table 3.

**Table 3 Production evaluation of bivalent nanobodies**

| **Bivalent nanobody ID** | **Bivalent nanobody structure** | **Yeast expression production (g/L)** |
|---|---|---|
| MY9431 | MY9286-GS20-MY9293 | 6.0 |
| MY9438 | MY9291-GS20-MY9292 | 2.0 |
| MY9439 | MY9291-GS20-MY9293 | 2.0 |
| MY9455 | MY9292-GS20-MY9286 | 2.0 |
| MY9456 | MY9292-GS20-MY9290 | 2.0 |
| MY9457 | MY9292-GS20-MY9291 | 2.0 |
| MY9460 | MY9292-GS20-MY9292 | 3.0 |
| MY9461 | MY9292-GS20-MY9293 | 5.0 |
| MY9464 | MY9293-GS20-MY9286 | 2.0 |
| MY9466 | MY9293-GS20-MY9291 | 2.0 |
| MY9470 | MY9293-GS20-MY9293 | 3.0 |

### Example 5 Activity evaluation of bivalent nanobodies

The above 11 humanized bivalent nanobodies were purified from the *Pichia pastoris* fermentation supernatant for the functional activity evaluation using HEK-Blue^{™} IL-4/IL-13 cells.

The results are shown in Table 4. The bivalent nanobodies demonstrated significantly enhanced activity, outperforming both Control antibody 1 (derived from patent WO2021116182A1) and Control antibody 2 (Lebrikizumab).

**Table 4 Functional activity evaluation of bivalent humanized antibodies**

| **Antibody ID** | **ActivityIC₅₀ (µg/mL)** | **Control antibody 1 IC₅₀ (µg/mL)** | **Control antibody 2 IC₅₀ (µg/mL)** |
|---|---|---|---|
| MY9431 | 0.0014 | 0.0050 | 0.0043 |
| MY9438 | 0.0454 | 0.0050 | 0.0043 |
| MY9439 | 0.0014 | 0.0050 | 0.0043 |
| MY9455 | 0.0014 | 0.0050 | 0.0043 |
| MY9456 | 0.0796 | 0.0051 | 0.0041 |
| MY9457 | 0.0015 | 0.0051 | 0.0041 |
| MY9460 | 0.3599 | 0.0051 | 0.0041 |
| MY9461 | 0.1308 | 0.0051 | 0.0041 |
| MY9464 | 0.0029 | 0.0040 | 0.0040 |
| MY9466 | 0.0038 | 0.0040 | 0.0040 |
| MY9470 | 1.7501 | 0.0040 | 0.0040 |

### Example 6 Construction of long-acting nanobodies

To confer favorable in vivo half-life characteristics to the above-mentioned nanobodies, an anti-human serum albumin nanobody (HuNb3-11) was incorporated. Various structural configurations of long-acting nanobodies were constructed, followed by evaluation of their production yield and biological activity. The preparation and evaluation methods were the same as in Example 4.

The evaluation results of its structure and expression production are shown in Table 5.

**Table 5 Production evaluation of long-acting nanobodies**

| **Nanobody ID** | **Antibody structure** | **Yeast expression production (g/L)** |
|---|---|---|
| MY9208 | MY9286-GS20-MY9293-GS20-HSA HuNb3-11 | 2.0 |
| MY9217 | MY9286-GS20-HSA HuNb3-11-GS20-MY9293 | 5.0 |
| MY9218 | MY9293-GS20-HSA HuNb3-11-GS20-MY9286 | 1.0 |
| MY9219 | HSA HuNb3-11-MY9286-GS20-MY9293-GS20 | 4.0 |

**Table 6 Amino acid sequence and nucleotide sequence**

| Nanobody region | MY9286 serial number | MY9293 serial number | HSA HuNb3-11 serial number |
|---|---|---|---|
| | (SEQ ID NO: ) | (SEQ ID NO: ) | (SEQ ID NO: ) |
| FR1 | 1 | 10 | 19 |
| CDR1 | 2 | 11 | 20 |
| FR2 | 3 | 12 | 21 |
| CDR2 | 4 | 13 | 22 |
| FR3 | 5 | 14 | 23 |
| CDR3 | 6 | 15 | 24 |
| FR4 | 7 | 16 | 25 |
| Complete amino acid sequence | 8 | 17 | 26 |
| Complete nucleotide sequence | 9 | 18 | 27 |

### Example 7 Activity evaluation of long-acting nanobodies

MY9217 and MY9219 were selected for IL-13 functional activity evaluation and serum albumin binding activity evaluation. The trivalent nanobodies purified from the fermentation broth were first subjected to IL-13 functional activity evaluation, using the same detection method as described in Example 2.

The results are shown in Table 2. The results show that both nanobodies effectively blocked IL-13-induced SEAP release in HEK-Blue^{™} IL-4/IL-13 cells, with activity superior to Control antibody 2 (Lebrikizumab, Roche). In addition, MY9217 and MY9219 exhibited effective binding to human serum albumin, with binding activity exceeding that of Control antibody 3 (HuNb3-11).

**Table 7 Activity evaluation of anti-IL-13 long-acting nanobodies**

| **Antibody ID** | **IL-13 functional activity IC₅₀ (µg/mL)** | **HSA binding activity EC₅₀ (µg/mL)** | |
|---|---|---|---|
| | | **pH 5.5** | **pH 7.4** |
| MY9217 | 0.0029 | 0.0953 | 0.0648 |
| MY9219 | 0.0024 | 0.0648 | 0.0467 |
| Control antibody 2 | 0.0076 | / | / |
| Control antibody 3 | / | 0.1639 | 0.1099 |

**Table 8 Amino acid sequence and nucleotide sequence of nanobodies MY9217 and MY9219**

| Antibody region | MY9217 serial number | MY9219 serial number |
|---|---|---|
| | (SEQ ID NO_{:} ) | (SEQ ID NO_{:} ) |
| Complete amino acid sequence | 29 | 31 |
| Complete nucleotide sequence | 30 | 32 |

### Example 8 Species binding activity

The binding activities of candidate nanobodies toward human IL-13 and cynomolgus monkey IL-13 were evaluated by ELISA. Human IL-13 and cynomolgus monkey IL-13 proteins were separately coated onto ELISA plates (4°C overnight). After blocking, serially diluted test nanobodies MY9217 and MY9219 were added and incubated at 37°C for 1 hour. This was followed by addition of diluted HRP-conjugated goat anti-nanobody polyclonal antibody (1: 2000 dilution) with 1-hour incubation at 37°C. Color development was initiated with TMB substrate and terminated with H₂SO₄ before absorbance measurement.

Results presented in Figure 1 demonstrate that both MY9217 and MY9219 simultaneously bind human IL-13 and cynomolgus monkey IL-13.

All references cited in this invention are incorporated herein by reference as if each reference were individually indicated for incorporation. In addition, it should be understood that after reading the above teachings of the present invention, those skilled in the art can make various changes or modifications to the present invention, and these equivalent forms also fall within the scope defined by the claims attached to the present application.

## Claims

1. An anti-IL-13 nanobody, wherein the complementary determining regions (CDRs) of the VHH chain in the nanobody are one or more selected from the group consisting of:
(1) CDR1 shown in SEQ ID NO: 2, CDR2 shown in SEQ ID NO: 4, and CDR3 shown in SEQ ID NO: 6; and
(2) CDR1 shown in SEQ ID NO: 11, CDR2 shown in SEQ ID NO: 13, and CDR3 shown in SEQ ID NO: 15.

2. The anti-IL-13 nanobody according to claim 1, wherein the VHH chain of the nanobody further comprises framework regions (FRs), and the framework regions (FRs) are one or more selected from the group consisting of:
(1) FR1 shown in SEQ ID NO: 1, FR2 shown in SEQ ID NO: 3, FR3 shown in SEQ ID NO: 5, and FR4 shown in SEQ ID NO: 7; and
(2) FR1 shown in SEQ ID NO: 10, FR2 shown in SEQ ID NO: 12, FR3 shown in SEQ ID NO: 14, and FR4 shown in SEQ ID NO: 16.

3. The anti-IL-13 nanobody according to claim 1, wherein the amino acid sequence of the VHH chain of the nanobody is selected from the group consisting of: SEQ ID NO: 8, SEQ ID NO: 17, and a combination thereof.

4. An anti-IL-13 antibody, which is directed against an IL-13 epitope and has the anti-IL-13 nanobody according to claim 1.

5. The anti-IL-13 antibody according to claim 1, which comprises one or more anti-IL-13 nanobodies.

6. The anti-IL-13 antibody according to claim 4 or 5, wherein the anti-IL-13 antibody comprises a monomer, a diabody (bivalent antibody), a tetrabody (tetravalent antibody), and/or a multibody (multivalent antibody).

7. A polynucleotide encoding a protein selected from the group consisting of: the anti-IL-13 nanobody according to claim 1 or the anti-IL-13 antibody according to claim 4.

8. An expression vector, which contains the polynucleotide according to claim 7.

9. A host cell, which contains the expression vector according to claim 8, or has the polynucleotide according to claim 7 integrated into its genome.

10. A method for producing an anti-IL-13 nanobody, comprising the steps of:
(a) culturing the host cell according to claim 9 under conditions suitable for producing nanobodies, thereby obtaining a culture containing the anti-IL-13 nanobody;
(b) isolating or recovering the anti-IL-13 nanobody from said culture; and
(c) optionally, purifying and/or modifying the anti-IL-13 nanobody obtained in Step (b).

11. An immunoconjugate comprising:
(a) the anti-IL-13 nanobody according to claim 1, or the anti-IL-13 antibody according to claim 4; and
(b) a coupling moiety selected from the group consisting of: a detectable label, a drug, a toxin, a cytokine, a radionuclide, an enzyme, a gold nanoparticle/nanorod, a nanomagnetic particle, a viral coat protein or VLP, and a combination thereof.

12. A multispecific antibody comprising: the anti-IL-13 nanobody according to claim 1, or the anti-IL-13 antibody according to claim 4.

13. A recombinant protein having:
(i) the anti-IL-13 nanobody according to claim 1, or the anti-IL-13 antibody according to claim 4; and
(ii) an optional tag sequence assisting expression and/or purification.

14. A pharmaceutical composition comprising:
(i) the anti-IL-13 nanobody according to claim 1, the anti-IL-13 antibody according to claim 4, or the immunoconjugate according to claim 11, or the multispecific antibody according to claim 12, or the recombinant protein according to claim 13; and
(ii) a pharmaceutically acceptable carrier.

15. Use of the anti-IL-13 nanobody according to claim 1, the anti-IL-13 antibody according to claim 4, or the immunoconjugate according to claim 11, or the multispecific antibody according to claim 12, or the recombinant protein according to claim 13, or the pharmaceutical composition according to claim 14, for:
(a) preparing drugs for preventing and/or treating IL-13-related diseases; and/or
(b) preparing reagents, assay plates, or kits for detecting IL-13.
